# EUROPEAN PATENT SPECIFICATION

(11) **EP 0 797 971 B1**
(45) Date of publication and mention of the grant of the patent: **02.11.2000**
(21) Application number: 97104882.2
(22) Date of filing: 21.03.1997
(51) Int. Cl.: A61F 13/20, D04H 1/54

(54) **Method and device for the shaping of wadding products**
Verfahren und Vorrichtung zur Formgebung von Watteprodukten
Méthode et dispositif pour la mise en forme de produits ouatés

(30) Priority: 22.03.1996 DE 19611469
(43) Date of publication of application: 01.10.1997
(73) Proprietor: JOHNSON & JOHNSON GmbH, 40474 Düsseldorf (DE)
(72) Inventor: Schoelling, Hans-Werner, 58252 Ennepetal (DE)
(74) Representative: Groening, Hans Wilhelm, Dipl.-Ing.

(56) References cited:
- EP-A- 0 611 562
- DE-A- 3 804 222
- US-A- 3 998 031
- US-A- 4 498 218

## Description

The invention relates to a method and device for the shaping of wadding products or the like, especially of tampons.

It is known that wadding products, for example tampons, ear buds or the like, used for bodily hygiene have a specific shape. Moreover, these wadding products can consist either of pure cotton or of mixed fibres or of a plastic/natural-fibre mixture. These wadding products are brought to the desired shape either by rolling or by applying a shaping tool. Thus, for example, a shaping tool having a recess is applied to the tip of a tampon, the recess having the shape to be imparted to the tampon tip.

The tampon is pressed under pressure into the die and the heated die transfers the contour of the recess to the tampon tip. The shaping tool has a different temperature depending on the wadding mixture. After the tampon has been introduced into the recess of the shaping tool, the tampon is pressed under pressure onto the die, with the result that the individual fibres of the wadding product are highly compacted in the pressing region and a smooth surface is ironed on in the region of the outer surface (see document DE-A-3 804 222).

It has been shown that this method gives good results when no pronounced changes in cross-section have to be made.

The object on which the invention is based is to provide a further method for the shaping of wadding products and/or a further device for the shaping of wadding products.

In a method of the type mentioned in the introduction, this object is achieved in that the preshaped wadding product is introduced at least in a portion into a shaping tool, and the wadding product is kept under pressure in the tool, that portion of the wadding product located in the tool being subjected to ultrasonic processing.

The invention is described below with reference to a tampon, although the invention is not to be restricted to this example of use, but is to embrace all wadding or wadding-like products.

According to the invention, the tampon region to be shaped is shaped by means of ultrasound, as a result of which the following advantageous effects are achieved:

By virtue of the action of ultrasound and, if appropriate, simultaneously of a pressing force on the tip of the tampon, heat is generated in this region. Moreover, the ultrasonic vibrations cause the individual fibres of the tampon to flow slightly, so that these slide off easily on one another, with the result that the flow behaviour and therefore the shaping behaviour of the wadding tip are influenced in a positive way. Furthermore, the ultrasonic vibrations reduce the friction between the wall of the tool and the tampon tip located in the tool. The heat generated by the ultrasonic vibration gives rise to an ironing or smoothing effect.

On account of the better flow behaviour of the individual fibres, drastic changes in cross-section can also be shaped, for example a paraboloidal head on a tampon.

According to a preferred feature of the invention, heat is supplied to the tampon before, during and/or after the ultrasonic processing. Thus, for example, a compaction of the surface can be achieved by pressing the tampon further into the ultrasonic tool and by the simultaneous or subsequent supply of heat. Furthermore, this surface has optimum smoothness. As a rule, the desired and necessary heat has already been generated as a result of the ultrasonic shaping.

It has been shown that optimum results are achieved when the ultrasonic waves are introduced into the tampon in the axial direction of the tampon, that is to say orthogonally to the receiving orifice of the processing tool.

Moreover, the object mentioned in the introduction is achieved by means of a device which has a guideholder, receiving the shank of the tampon, and a shaping tool capable of being applied to the tip of the tampon, the shaping tool being designed as sonotrode and the sonotrode having a recess corresponding to the desired shapes of the tip of the tampon. Thus, the tampon can be shaped into the finished product in one operation. The preshaping of the tampon merely involves bringing the latter into a cylindrical or approximately cylindrical shape. By energizing the sonotrode in the axial direction of the tampon, the tip is brought to the desired shape, irrespective of the initial shape.

Advantageously, in this case, the guideholder is movable in the direction of the sonotrode or else the sonotrode is movable in the direction of the guideholder or both are movable relative to one another.

In one embodiment, there is provision for equipping the guideholder with a sliding means which displaces the tampon in the guideholder. Via this sliding means, the tampon can additionally be pressed into the sonotrode, for example after the sonotrode has been applied, so that, in addition to the ultrasonic waves, pressure is also exerted on the tampon and therefore on the tampon tip. It is also possible, however, to push in the tampon further via the sliding means and put it under pressure only after the ultrasonic processing, so that the fibres located on the surface of the tampon tip are compacted.

Preferably, the sonotrode is capable of being heated or cooled. The sonotrode can thereby assume a temperature which is optimum for the shaping operation, so that the fibres are additionally also ironed.

In an exemplary embodiment, an element absorbing ultrasonic vibrations is provided between the free end face of the sonotrode and that end face of the guideholder which faces the sonotrode. During the ultrasonic processing, this element allows a guideholder to bear over its entire area on the sonotrode, without the vibrations of the sonotrode being transmitted to the guideholder. Energy is therefore introduced solely into the tampon. In a preferred exemplary embodiment, the sonotrode has a vibration frequency of up to 35 kHz and/or an amplitude of 50 to 100 µm, especially of 80 µm. Optimum processing of the tip is achieved by means of these values.

For the simultaneous processing of a plurality of tampons, the sonotrode has a plurality of recesses. In this case, the sonotrode is designed so that the introduction of energy into the individual tampons is distributed uniformly.

Further advantages, features and particulars of the invention emerge from the following description which, with reference to the drawing, represents in detail an especially preferred exemplary embodiment. At the same time, the features shown in the drawing and mentioned in the claims of the description can be essential to the invention each individually in themselves or in any combination.
In the drawing:
- Figure 1: shows a shaping device according to the invention;
- Figure 2: shows a ready-shaped wadding product;
- Figure 3: shows a device for the shaping of wadding products according to the prior art.

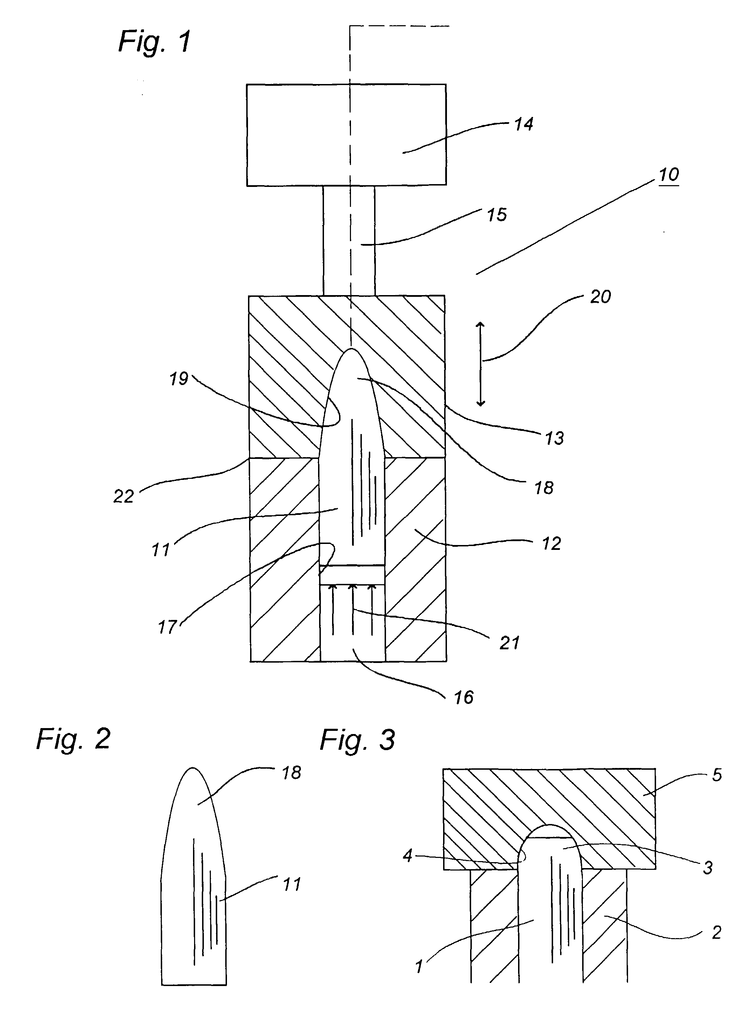

Figure 3 shows a conventional shaping device, in which a tampon 1 having an essentially circular cross-section is held in a guideholder 2. By means of this guideholder 2, the tampon 1, the tip 3 of which projects beyond the guideholder 2, is introduced into a recess 4 of a heated shaping tool 5. The recess 4 has the shape of the desired tampon tip to be produced. The tampon 1 is introduced under pressure into this recess 4, with the result that the fibres in the tip region are compacted. On account of the continuously decreasing cross-section, the friction of the tampon fibres on the surface of the recess 4 increases and the individual tampon fibres are compacted in the tip region, so that the internal friction also rises sharply. It has been shown that the outermost tampon tip is shaped only insufficiently or, as shown in Figure 3, not at all.

Figure 1 shows a shaping device, designated as a whole by 10, which likewise has a guideholder 12 for the tampon 11 to be shaped. This guideholder 12 is assigned a sonotrode 13 with converter 14 and transformer 15. Moreover, the guideholder 12 has a sliding means 16 which is arranged displaceably in the orifice 17 of the guideholder 12.

The tampon 11 to be shaped is inserted into this orifice 17, so that its tip 18 to be shaped projects beyond the guideholder 12. Subsequently, either the guideholder 12 is brought nearer to the sonotrode 13 and/or the sonotrode 13 is brought nearer to the guideholder 12, with the result that the tip 18 is introduced into a recess 19 of the sonotrode 13. The sonotrode 13 is then set in vibration, as indicated by the double arrow 20. At the same time, the surface friction between the recess 19 and fibres of the tampon 11 and the mutual friction of the individual fibres are greatly reduced, so that the tip 18 of the tampon 11 adapts to the shape of the recess 19 and assumes this shape. Simultaneously, heat is supplied to the tampon tip 18 via the sonotrode 13. Either this heat can be introduced externally or it is generated during the ultrasonic processing. Furthermore, during the ultrasonic processing, the tampon 11 can be pushed further by the sliding means 16 and, as indicated by arrows 21, pressure can be exerted on the tampon 11, so that the tip 18 is pressed into the recess 19. The sliding means 16 can be actuated during ultrasonic processing and/or also after the conclusion of ultrasonic processing, which lasts from between 0.2 and 5 seconds, especially 0.5 seconds. An element 22 absorbing ultrasonic vibrations is located between the guideholder 12 and the sonotrode 13, so that, even during ultrasonic processing, the guideholder 12 can touch the sonotrode 13 and nevertheless the entire sound energy is introduced into the tampon 11 or its tip 18. The pressing of the fibres of the tampon tip 18 onto the hot surface of the recess 19 gives rise to an ironing effect and to a compaction of the fibres.

As shown in Figure 2, tampons 11 which have, for example, a paraboloidal tip can be produced by means of this method according to the invention and this device according to the invention. In contrast to a hemispherical tip, in the case of this paraboloidal tip 18 a cross-section of the tampon 11 changes over a wide range, and for this reason shaping according to the prior art presents problems.

## Claims

1. Method for the shaping of wadding products or the like, especially of tampons (11), having the following method steps:
- the preshaped wadding product is introduced at least in a portion into a shaping tool,
and
- the wadding product is kept under pressure in the tool,
characterized
in that that portion of the wadding product located in the tool is subjected to ultrasonic processing.

2. Method according to Claim 1, characterized in that the shape of the ultrasonic tool is imparted to the portion of the wadding product by means of ultrasound.

3. Method according to one of the preceding claims, characterized in that heat is supplied to the wadding product before, during and/or after the ultrasonic processing.

4. Method according to one of the preceding claims, characterized in that the wadding product is introduced further into the tool during and/or after the ultrasonic processing.

5. Method according to one of the preceding claims, characterized in that the surface of that portion of the wadding product processed by means of ultrasound is smoothed during the ultrasonic processing.

6. Method according to one of the preceding claims, characterized in that the surface of that portion of the wadding product processed by means of ultrasound is compacted during and/or after the ultrasonic processing.

7. Method according to one of the preceding claims, characterized in that the ultrasonic waves are introduced in the axial direction of the wadding product.

8. Device for the shaping of wadding products or the like, especially of tampons (11), especially according to one of the preceding claims, having a guide holder (12), receiving the shank of the wadding product, and a shaping tool capable of being applied to the tip of the wadding product, characterized in that the shaping tool is designed as a sonotrode (13) and the sonotrode (13) has a recess (19) corresponding to the desired shapes of the tip (18) of the wadding product.

9. Device according to Claim 8, characterized in that the sonotrode (13) is energized in the axial direction of the wadding product or orthogonally to the orifice cross-section of the recess (19).

10. Device according to Claim 8 or 9, characterized in that the guide holder (12) is movable in the direction of the sonotrode (13) and/or conversely.

11. Device according to one of Claims 8 to 10, characterized in that the guide holder (12) is equipped with a sliding means (16) displacing the wadding product in the guideholder (12).

12. Device according to one of Claims 8 to 11, characterized in that the sonotrode (13) is capable of being heated or cooled.

13. Device according to one of Claims 8 to 12, characterized in that an element absorbing ultrasonic vibrations is provided between the free end face of the sonotrode (13) and that end face of the guideholder (12) facing the sonotrode (13).

14. Device according to one of Claims 8 to 13, characterized in that the sonotrode (13) has a vibration frequency of up to 35 kHz and/or an amplitude of 50 to 150 µm, especially of 80 µm.

15. Device according to one of Claims 8 to 14, characterized in that the sonotrode (13) has a plurality of recesses (19).

## Patentansprüche

1. Verfahren zum Umformen von Watteprodukten oder dergleichen, insbesondere von Tampons (11), mit folgenden Verfahrensschritten:
- das vorgeformte Watteprodukt wird wenigstens abschnittsweise in ein Umformwerkzeug eingeführt, und
- das Watteprodukt wird unter Druck im Werkzeug gehalten,
**dadurch gekennzeichnet**,
daß der im Werkzeug sich befindende Abschnitt des Watteprodukts einer Ultraschallbearbeitung unterzogen wird.

2. Verfahren nach Anspruch 1, dadurch gekennzeichnet, daß dem Abschnitt des Watteprodukts mittels Ultraschall die Form des Ultraschallwerkzeugs angeformt wird.

3. Verfahren nach einem der vorhergehenden Ansprüche, dadurch gekennzeichnet, daß vor, während und/oder nach der Ultraschallbearbeitung dem Watteprodukt Wärme zugeführt wird.

4. Verfahren nach einem der vorhergehenden Ansprüche, dadurch gekennzeichnet, daß während und/oder nach der Ultraschallbearbeitung das Watteprodukt weiter in das Werkzeug eingeführt wird.

5. Verfahren nach einem der vorhergehenden Ansprüche, dadurch gekennzeichnet, daß die Oberfläche des mittels Ultraschall bearbeiteten Abschnitts des Watteprodukts während der Ultraschallbearbeitung geglättet wird.

6. Verfahren nach einem der vorhergehenden Ansprüche, dadurch gekennzeichnet, daß die Oberfläche des mittels Ultraschall bearbeiteten Abschnitts des Watteprodukts während und/oder nach der Ultraschallbearbeitung verdichtet wird.

7. Verfahren nach einem der vorhergehenden Ansprüche, dadurch gekennzeichnet, daß die Ultraschallwellen in Achsrichtung des Watteprodukts eingeleitet werden.

8. Vorrichtung zum Umformen von Watteprodukten oder dergleichen, insbesondere von Tampons (11), insbesondere nach einem der vorhergehenden Ansprüche, mit einem den Schaft des Watteprodukts aufnehmenden Führungshalter (12) und einem auf die Spitze des Watteprodukts aufsetzbaren Umformwerkzeug, dadurch gekennzeichnet, daß das Umformwerkzeug als Sonotrode (13) ausgebildet ist und die Sonotrode (13) eine der gewünschten Formen der Spitze (18) des Watteprodukts entsprechende Ausnehmung (19) aufweist.

9. Vorrichtung nach Anspruch 8, dadurch gekennzeichnet, daß die Sonotrode (13) in Achsrichtung des Watteprodukts bzw. orthogonal zum Öffnungsquerschnitt der Ausnehmung (19) angeregt wird.

10. Vorrichtung nach Anspruch 8 oder 9, dadurch gekennzeichnet, daß der Führungshalter (12) in Richtung der Sonotrode (13), und/oder umgekehrt, verfahrbar ist.

11. Vorrichtung nach einem der Ansprüche 8 bis 10, dadurch gekennzeichnet, daß der Führungshalter (12) mit einer das Watteprodukt im Führungshalter (12) verlagernden Schiebeeinrichtung (16) versehen ist.

12. Vorrichtung nach einem der Ansprüche 8 bis 11, dadurch gekennzeichnet, daß die Sonotrode (13) beheizbar oder kühlbar ist.

13. Vorrichtung nach einem der Ansprüche 8 bis 12, dadurch gekennzeichnet, daß zwischen der freien Stirnfläche der Sonotrode (13) und der der Sonotrode (13) zugewandten Stirnfläche des Führungshalters (12) ein Ultraschallschwingungen absorbierendes Element vorgesehen ist.

14. Vorrichtung nach einem der Ansprüche 8 bis 13, dadurch gekennzeichnet, daß die Sonotrode (13) eine Schwingfrequenz von bis zu 35 kHz und/oder eine Amplitude von 50 bis 150 µm, insbesondere von 80 µm, aufweist.

15. Vorrichtung nach einem der Ansprüche 8 bis 14, dadurch gekennzeichnet, daß die Sonotrode (13) mehrere Ausnehmungen (19) aufweist.

## Revendications

1. Procédé de mise en forme de produits ouatés ou analogues, spécialement de tampons (11), comportant les étapes de procédé suivantes consistant à :
- introduire le produit ouaté préformé au moins en partie dans un outil de mise en forme,
et
- maintenir le produit ouaté sous pression dans l'outil,
caractérisé
en ce que la partie du produit ouaté positionnée dans l'outil est soumise à un traitement aux ultrasons.

2. Procédé selon la revendication 1, caractérisé en ce que la forme de l'outil à ultrasons est transmise à la partie du produit ouaté par l'intermédiaire d'ultrasons.

3. Procédé selon l'une quelconque des revendications précédentes, caractérisé en ce que de la chaleur est fournie au produit ouaté avant, pendant et/ou après le traitement aux ultrasons.

4. Procédé selon l'une quelconque des revendications précédentes, caractérisé en ce que le produit ouaté est introduit plus loin dans l'outil pendant et/ou après le traitement par ultrasons.

5. Procédé selon l'une quelconque des revendications précédentes, caractérisé en ce que la surface de la partie du produit ouaté traitée par l'intermédiaire d'ultrasons est lissée lors du traitement aux ultrasons.

6. Procédé selon l'une quelconque des revendications précédentes, caractérisé en ce que la surface de la partie du produit ouaté traitée par l'intermédiaire d'ultrasons est rendue compacte pendant et/ou après le traitement aux ultrasons.

7. Procédé selon l'une quelconque des revendications précédentes, caractérisé en ce que les ondes ultrasonores sont introduites dans la direction axiale du produit ouaté.

8. Dispositif pour la mise en forme de produits ouatés ou analogues, spécialement de tampons (11), spécialement selon l'une quelconque des revendications précédentes, ayant un support de guide (12), recevant la tige du produit ouaté, et un outil de mise en forme capable d'être appliqué à la pointe du produit ouaté, caractérisé en ce que l'outil de mise en forme est conçu en tant qu'électrode ultrasonique (13) et l'électrode ultrasonique (13) a un évidement (19) correspondant aux formes voulues de la pointe (18) du produit ouaté.

9. Dispositif selon la revendication 8, caractérisé en ce que l'électrode ultrasonique (13) est mise sous tension dans la direction axiale du produit ouaté ou orthogonalement à la coupe d'orifice de l'évidement (19).

10. Dispositif selon la revendication 8 ou 9, caractérisé en ce que le support de guide (12) est mobile dans la direction de l'électrode ultrasonique (13) et/ou réciproquement.

11. Dispositif selon l'une quelconque des revendications 8 à 10, caractérisé en ce que le support de guide (12) est muni de moyens de coulissement (16) déplaçant le produit ouaté dans le support de guide (12).

12. Dispositif selon l'une quelconque des revendications 8 à 11, caractérisé en ce que l'électrode ultrasonique (13) peut être chauffée ou refroidie.

13. Dispositif selon l'une quelconque des revendications 8 à 12, caractérisé en ce qu'un élément absorbant des vibrations ultrasonores est agencé entre la face d'extrémité libre de l'électrode ultrasonique (13) et la face d'extrémité du support de guide (12) dirigée vers l'électrode ultrasonique (13).

14. Dispositif selon l'une quelconque des revendications 8 à 13, caractérisé en ce que l'électrode ultrasonique (13) a une fréquence de vibration allant jusqu'à 35 kHz et/ou une amplitude de 50 à 150 µm, spécialement de 80 µm.

15. Dispositif selon l'une quelconque des revendications 8 à 14, caractérisé en ce que l'électrode ultrasonique (13) comporte une pluralité d'évidements (19).
